# EUROPEAN PATENT SPECIFICATION

(11) **EP 3 181 202 B1**
(45) Date of publication and mention of the grant of the patent: **18.09.2019**
(21) Application number: 15200854.6
(22) Date of filing: 17.12.2015
(51) Int. Cl.: A63F 9/00, A63B 24/00, G08G 1/07, G09B 19/00, G16H 40/60, G16H 20/30, A63B 69/00, A63B 71/06, A61B 5/16

(54) **A DEVICE FOR EXERCISING A REACTION TO STIMULI AND THE RELATED METHOD**
VORRICHTUNG ZUM ÜBEN EINER REAKTION AUF REIZE UND ENTSPRECHENDES VERFAHREN
DISPOSITIF POUR EXERCER UNE RÉACTION À DES STIMULI ET PROCÉDÉ ASSOCIÉ

(43) Date of publication of application: 21.06.2017
(73) Proprietor: Ledsreact BVBA, 8560 Wevelgem (BE)
(72) Inventor: Vercauteren, Koen, 8560 Wevelgem (BE); Segers, Mathias, 8560 Wevelgem (BE)
(74) Representative: IP HILLS NV

(56) References cited:
- WO-A1-2009/039661
- WO-A1-2013/071408
- GB-A- 2 502 083
- US-A1- 2008 051 228
- US-A1- 2015 065 273
- US-B1- 8 777 818

## Description

### Field of the Invention

The present invention generally relates to sports training systems and in particular to developing and training reaction skills to stimuli.

### Background of the Invention

In many sports, strength, dexterity and fast reflexes are valuable attributes for athletes. Reaction time to stimuli can be trained in order to minimize the time between the generation of the stimuli and the reaction of the athletes. Training one's reaction time is useful for example in football, in basketball, in athletics, in swimming, in volleyball, in handball, in tennis, in baseball and in cricket, in American football, and in any other sport where running trajectories or reactions must be adapted in function of stimuli, such as the sound of a starting pistol in athletics and in swimming, or the shot of a penalty in football, or passes, or the shot of a drop shot in tennis. Examples of apparatuses and systems to train one's reaction time are for example described in GB2502083, WO2009/039661, US2015/0065273, US2008/0051228, US8777818 and WO2013/071408. WO2013/071408 describes a stimulant target unit 10. According to paragraphs [8] and [26] of WO2013/071408, each stimulant target unit 10 comprises a light source 20, a proximity sensor to provide an output distance between the unit and an external object and feedback means to inform the user that the unit has been actuated. The light source 20 of each stimulant target unit 10 is an annular arrangement of light emitting diodes 20 which provides a uniform light in the shape of a ring, as described in paragraph [54] of WO2013/071408. The annular array of light emitting diodes 20 provides originating stimulus by being turned on or may also provide feedback stimulus by being turned off, as described in paragraph [56] of WO2013/071408. In other words, each stimulant target unit 10 comprises one single indicator comprising a plurality of light emitting diodes 20 which all turn on or off simultaneously. Additionally, a controller external to the stimulant target unit 10 may be connected to the light source, to the proximity sensor, and to the feedback means of a stimulant target unit 10 of WO2013/071408. In other words, the external controller WO2013/071408 is not comprised in the housing of a stimulant target unit 10 of WO2013/071408. The controller of WO2013/071408 "(i) signals the stimulant target unit to illuminate, (ii) registers user reaction to the illumination in bringing a body part or other object to within a selected distance of the proximity sensor, and (iii) thereafter activates the feedback means to alert the user that the stimulant target unit has been actuated", as described in paragraph [25] of WO2013/071408. In other words, the external controller of WO2013/071408 instructs a stimulant target unit 10 to generate illumination before the stimulant target unit 10 detects a presence of a body part within a selected distance of its proximity sensor. In WO2013/071408, first, the light source 20 is illuminated, and then the proximity sensor of the corresponding stimulant target unit 10 detects presence of a body part within a selected distance of the proximity sensor. Additionally, a plurality of stimulant target units 10 of WO2013/071408 may be utilized as a part of a system for exercising running manoeuvres, e.g. to train football players, as described in paragraph [61] of WO2013/071408. Such a system is depicted on Figure 12 of WO2013/071408. The system of WO2013/071408 comprises a plurality of stimulant target units 10 and further comprises the external system controller 200, as explained in paragraph [26] of WO2013/071408. Paragraph [75] of WO2013/071408 describes that the system controller 200 instructs (i) which stimulant target units 10 should light up, (ii) the proximity distance to register a hit for the stimulant target unit in question and (iii) the time delay to the activation of the next stimulant target unit 10 in the sequence. Similarly, the system controller 200 of WO2013/071408 first instructs a first stimulant target unit 10 to generate illumination before the first stimulant target unit 10 detects a presence of a body part within a selected distance of its proximity sensor. This sequence is further repeated during running manoeuvres where the system controller 200 of WO2013/071408 consequently instructs a second stimulant target unit 10 to generate illumination before the second stimulant target unit 10 detects a presence of a body part within a selected distance of its proximity sensor, etc.. US8777818 describes a device which moves in response to sensed change in proximity of a user of the device. In other words, the device described by US8777818 counters the action of the user when the user comes in proximity with the device.

WO2007/142588A1 by IC Control Media & Sport AB, published on December 13^{th} 2007, discloses a system for exercising a human reaction time to stimuli in connection with sports activities and/or rehabilitation purposes. The system comprises a first means for generating stimuli for a user to react and a second means for controlling the stimuli. The first means comprises one or more light emitting elements and may also comprise a device for sound emission, activated simultaneously with the light emitting elements. The first means is activated by a control means. When a stimulus is generated by the first means, the user can react by making a movement. For example, the user can be moving an arm or a leg into the beam of light, thereby interrupting the light beam. The user's reaction is then registered by a movement sensor, and a reaction time of the user is communicated to the control means by a wireless connection means. The control means is operated by the user who can choose randomized stimuli generation or pre-programmed sequences of stimuli. The system may be arranged to create a three-dimensional environment for training, for example by mounting a number of first means and of movement sensors on footed poles of different lengths and in several distances relative to the user.

The system disclosed in WO2007/142588A1 trains the human reaction time to a visual and/or audible stimulus. However, the system does not train the actual user's physical movement resulting from the visual stimulus. It is for example possible for a movement sensor to detect a movement of the user after the generation of a stimulus, even if the user is not facing the corresponding first means, even if the user does not move towards the first means or even if the user does not direct his movement towards the corresponding first means. It then becomes possible for the user to accidently interrupt the light beam, which threatens the relevancy of the measurements of his reaction time. Additionally, an athlete wishes to train an actual physical movement as a reaction to stimuli, such as for example a jump in basketball, a sudden change in running direction in football, a dive in swimming, etc., whereas the system disclosed in WO2007/142588A1 is not specifically implemented to train the actual physical movement. In addition, a user of the system of WO2007/142588A1 must be able to visually monitor all the first means of the three-dimensional environment in order to identify the first means generating the visual stimulus. The user's reaction time is then related to the speed of his eyesight and of his movement as reaction to the stimulus. For example, when the user is surrounded by first means, it may happen that a first means positioned in the back of the user generates a stimulus. The user must first turn around to be able to notice the first means. His reaction time then comprises the time needed by the user to notice the first means as well as the time needed to interrupt the light beam.

It is an objective of the present invention to disclose a device that overcomes the above identified shortcomings of existing solutions. More particularly, it is an objective to disclose a device for exercising reaction to stimuli that considers the actual user's movement, and that enables to more accurately determine a reaction time.

### Summary of the Invention

According to a first aspect of the present invention, the above defined objectives are realized by a device for exercising reaction to stimuli as claimed in claim 1.

The device according to the present invention only generates a stimulus when presence is detected by a proximity sensor. A user of the device can therefore accurately train his reaction to a stimulus as the user himself triggers the generation of the stimulus when his presence is detected in the proximal sensing range of a proximity sensor. The user therefore does not have to identify which device generates a stimulus, thereby ensuring that a real reaction time of the user is measured. Additionally, each stimulus is indicative for a different direction. Upon detection of presence, only one of the indicators of the device according to the present invention generates a stimulus. In other words, each stimulus corresponds to the indication of one respective direction that a person detected in the proximal sensing range of the proximity sensor must follow. The device according to the present invention therefore allows a user of the device to train a specific physical movement required to follow the respective direction associated with the respective stimulus. For example, the device may lie on the ground. A direction is then a direction along which the user must move, for example by walking, or running, or crawling, etc., i.e. translation movements in a plane parallel to the ground. This is for example useful to footballers, basketballers, athletes in athletics, etc. who must train an unexpected change of direction when running to for example defend against an opponent, to get to the ball, or who must train their reaction to a starting pistol, etc.. Alternatively, the device may for example be positioned along a vertical wall. A direction is then a direction along which the user must exercise a physical movement, for example by walking, or running, or jumping or bending, i.e. translation movements in a plane parallel to the vertical wall. This is for example useful to basketballers, athletes in athletics, swimmers, etc. who must train an unexpected change of direction when running to defend against an opponent, to get to the ball, or who must train their reaction to a starting pistol, etc.. The device according to the present invention hence generates a stimulus that must trigger a particular reaction from the user. The device hence enables to discriminate between correct and incorrect reaction to stimuli.

In accordance with the present invention, the device comprises two or more indicators, for example three, four, five, six, seven or eight indicators. The device is preferably round, but may alternatively be square, triangular, hexagonal, octagonal, etc. Only one indicator is positioned on each edge of the device. The indicators are respectively indicative for a left or a right direction and a straightforward direction, or a left or a right direction and a backward direction, or a left direction and a right direction. Each indicator may demonstrate a different colour, for example red, green, blue, yellow, white, etc., and to each respective direction is always associated the same colour. The indicators extend on the top the device, thereby being visible from the top of the device. Alternatively, the indicators extend along the sidewalls of the device, thereby being visible on the sides of the device. A stimulus is a continuous visual signal. Alternatively, a stimulus is a flashing visual signal, or a periodically blinking visual signal, etc. A proximity sensor is an infrared sensor. The proximal sensing range of the proximity sensor is defined as an area comprised between an outer sensing range boundary and the proximity sensor. The outer sensing range boundary corresponds to positions from which presence is detectable by the proximity sensor and for which the distance between the proximity sensor and the positions is the furthest allowable sensing distance for the proximity sensor. The outer sensing range boundary is for example defining a section of a circle or a circle with a radius corresponding to a furthest allowable sensing distance to the proximity sensor. The proximity sensing range extends for example 10 meters, 2 meters, or 1 meter, or 50 centimetres, or 40 centimetres, or 30 centimetres from the proximity sensor, i.e. the distance from which the device will generate stimuli. The furthest allowable sensing distance may be the furthest sensing distance of the proximity sensor. Alternatively, the furthest allowable sensing distance is programmed in the proximity sensor.

In accordance with the present invention, presence is human presence, and a user of the device is a human. Human presence may be detected by for example an infra-red proximity sensor. Alternatively, presence is animal presence, and a user of the device is an animal, for example presence of a horse, a dog, or any other animal. Animal presence may be detected by for the example an infra-red proximity sensor. Alternatively, presence is presence of an object, for example presence of a car such as a rally car, presence of a bike, presence of a kart, presence of a quad, or presence of any two-wheeled or four-wheeled vehicle, etc.. A user of the device is then a user of the object, for example a driver of a car, of a rally car, a cyclist, a kart rider, a quad rider, etc.. In accordance with the present invention, the device exercises reaction to stimuli, wherein the device comprises a proximity sensor adapted to detect human presence in a proximal sensing range of the proximity sensor, two or more indicators, each indicator being configured to generate a respective stimulus indicative for a respective direction, and a controller configured to instruct one of the two or more indicators to generate the respective stimulus for the respective direction upon detection of the human presence. Alternatively, the device exercises reaction to stimuli, wherein the device comprises a proximity sensor adapted to detect presence of an object in a proximal sensing range of the proximity sensor, two or more indicators, each indicator being configured to generate a respective stimulus indicative for a respective direction, and a controller configured to instruct one of the two or more indicators to generate the respective stimulus for the respective direction upon detection of presence of the object. Alternatively, the device exercises animal reaction to stimuli, wherein the device comprises a proximity sensor adapted to detect animal presence in a proximal sensing range of the proximity sensor, two or more indicators, each indicator being configured to generate a respective stimulus indicative for a respective direction, and a controller configured to instruct one of the two or more indicators to generate the respective stimulus for the respective direction upon detection of animal presence. This way, the device according to the present invention hence generates a stimulus that must trigger a particular reaction from an animal, or from a human exercising a sport, or from a human driving. The device hence enables to discriminate between correct and incorrect reaction to stimuli.

According to an optional embodiment, the device further comprises a reaction sensor adapted to:
- detect movement along the respective direction;
- determine a reaction time between generation of the respective stimulus and detection of the movement along the respective direction.

The reaction sensor determines a reaction time between the time at which a stimulus is generated and the time at which a movement along the corresponding respective direction is detected. The reaction sensor is therefore adapted to detect a movement in the proximity sensing range of the proximity sensor and is further adapted to determine the movement direction. When the direction along which movement is detected corresponds to the respective direction indicated by the respective generated stimulus, the reaction time of a user of the system is logged. This improves the accuracy of the determination of a reaction time of the user and allows to discriminate good reaction from bad reaction to stimuli. Additionally, the reaction time may be accessed and the progress may be monitored in function of the evolution of the reaction time.

According to an optional aspect of the invention, the proximity sensor and the reaction sensor are integrated in embodiments of the invention.

This way, the design of the device is simple, compact and the costs associated with the implementation of such a device are lowered.

According to an optional aspect, embodiments of the device comprise three indicators, respectively indicative for a left direction, a right direction and a straightforward direction.

This way, a single device can indicate three different directions. The three indicators are arranged in a triangle, for example an isosceles triangle, wherein each indicator extends on the top surface and/or the side of the device. When facing the device, the device may indicate a left direction, or a right direction or a straightforward direction. Alternatively, when facing the device, the device may indicate a left direction, a right direction or a backward direction. This way, a user of the device can train a lateral movement reaction to a stimulus indicative for a left direction or a right direction, or can train a movement reaction to a stimulus indicative for a straightforward direction. It is possible to train a reaction along one of three distinctive directions with a single device.

According to an optional aspect, embodiments of the device comprise four indicators, respectively indicative for a left direction, a right direction, a straightforward direction and a backward direction.

This way, a single device can indicate four different directions. The four indicators are arranged in a quadrilateral, for example a square, or a rectangle, or a rhombus, or a parallelogram, wherein each indicator extends on the top surface and/or the side of the device. When facing the device, the device may indicate a left direction, or a right direction, or a straightforward direction or a backward direction. This way, a user of the device can train a lateral movement reaction to a stimulus indicative for a left direction or a right direction, or can train a movement reaction to a stimulus indicative for a straightforward direction or a backward direction. It is possible to train a reaction along one of four distinctive directions with a single device.

Optionally, the indicators are light-emitting diodes.

The advantages to use light-emitting diodes, also referred to as LEDs, are multiple. LEDs demonstrate a relatively long useful lifetime of tens of thousands of hours of useful life. LEDs are also shock resistant, which makes them difficult to damage with external shock. LEDs demonstrate slow failure, and mostly fail by dimming over time, which maximizes the amount of time before which each indicator must be replaced. Additionally, LEDs are ideal for uses subject to frequent on-off cycling, and light up very quickly. LEDs are very small, and can be easily attached to printed circuit boards, while demonstrating a good efficiency, thereby lowering initial costs. LEDs are also available in several colours, without the need of a colour filter, such as red, blue, green, yellow, white, etc. LEDs can be engineered to demonstrate a wide viewing angle, and/or a high brightness, which ensures the visibility of the indicators in rainy and/or foggy environments. LEDs have a little impact on the battery, and demonstrate a long autonomy.

According to an optional aspect, to the respective direction is associated one colour of said light-emitting diodes.

This way, to each respective direction corresponds one colour of light-emitting diodes. In other words, a respective direction is always indicated by an indicator of the same colour, and this colour is different from the colour of the indicators indicating another respective direction. For example a straightforward direction may always be indicated by a blue light-emitting diode, a right direction may always be indicated by a red light-emitting diode, and a left direction may always be indicated by a green light-emitting diode. Alternatively, a straightforward direction may always be indicated by a green light-emitting diode, a right direction may always be indicated by a blue light-emitting diode, a left direction may always be indicated by a white light-emitting diode, and a backward direction may always be indicated by a red light-emitting diode. This way, a user of the device associates a colour of the indicator with a respective direction which must be followed and thereby more accurately identifies the respective direction in function of the colour of the indicator.

According to an embodiment, the device further comprises an auditory signal generator configured to generate an auditory stimulus indicative for the respective direction upon detection of the presence.

This way, each auditory stimulus is also indicative for the respective direction. An auditory stimulus may therefore be indicative for a right direction, a left direction, a straightforward direction or a backward direction. The simultaneity between the generation of the stimulus and the auditory stimulus facilitates noticing the stimulus. It enables to more accurately determine the actual reaction time of the user and it enables to train reaction to auditory stimuli which may be important for instance for runners, swimmers, etc..

According to an embodiment, the device further comprises a wireless receiver adapted to receive signals indicative for the respective direction.

This way, the stimuli of the indicators are generated in function of control signals received by the wireless receiver. A wireless receiver is for example a Wi-Fi receiver. Alternatively, the wireless receiver comprises an RF antenna. This enables a central unit to determine the respective direction, which is for instance desired if plural devices are used to train certain patterns.

According to an embodiment, the signals are randomly alternating between available directions.

This way, random patterns can be trained, as a result of which the next user or player cannot predict which stimuli will be generated by watching previous players do the exercise.

According to an embodiment, the signals follow a predetermined pattern of available directions.

This way, a user of the device and/or a trainer of the user of the device can pre-program a sequence of stimuli according to a predetermined pattern, in order to train a specific physical movement of the user or certain running patterns. The respective directions are pre-programmed, and the device receives signals indicative for the pre-programmed respective directions.

According to an embodiment, the device further comprises a wireless transmitter adapted to transmit the reaction time.

This way, the reaction time of a user of the device may be transmitted to another device, which may be adapted to store it. The reaction time of the user may then be retrieved and the user can monitor his progress in function of the evolution of the reaction time. Alternatively, the reaction time is wirelessly transmitted to a screen adapted to display the reaction time of the user. The screen may be a screen positioned on the field, or may for example be the screen of a computer, of a tablet, of a watch, or a personal training device, such as a smartphone, or a GPS, etc..

According to an embodiment, the device further comprises a wireless transceiver configured to connect the device with other similar devices, and to determine a relative position of the device versus the other devices.

This way, plural devices can be positioned, the devices interconnect automatically and each device becomes aware of neighbouring devices and of their relative positions and distances to the device.

According to an optional embodiment, the device further comprises a battery.

This way, the device is made portable. The device must not be connected with a wire to a power source, which allows a user to easily transport the compact device where necessary. Additionally, the battery ensures an autonomy of the device, which allows a user to train for example for several hours or for several days without needing to recharge the device.

According to a second aspect of the invention, there is provided a system for exercising reaction to stimuli as claimed in claim 15.

By positioning several devices according to the present invention on for example a football field, or a basketball field, or an American football field, a system comprising a plurality of the devices can generate a sequence of stimuli, each respective stimulus being indicative for a respective direction which must be followed by a user in training. A user of the system triggers himself the generation of each stimulus every time his presence is detected in the proximal sensing range of a proximity sensor of a device. The user therefore does not have to identify which device of the system is configured to generate a stimulus, thereby reducing the time between the generation of the stimulus and the detection of a reaction of the user to the stimulus. This way, the system according to the present invention sets up a path on for example the football field, or the basketball field, or the American football field, created by the stimuli generated by the devices, each device being configured to indicate a respective direction that the user must follow. The path is preferably continuous in the system. In other words, the plurality of devices is arranged on a field such that a stimulus generated by a device upon detection of a presence indicates a respective direction of another device of the system. The training of the user of the system thereby continues until the path leads to a device which does not indicate a respective direction, which corresponds to the end of the path. Preferably, the path comprises all the devices of the system, i.e. all the devices of the system generate a stimulus at least once. Alternatively, the path does not comprise one or more devices of the system, which therefore do not generate a stimulus. The devices may be equidistantly positioned on a field. Preferably, they may be positioned non-equidistantly on a field, thereby training the movement speed of the user between two consecutive devices. Additionally, a user of the system is able to train patterns of stimuli, sequences of reactions, etc. with the possibility to measure the time needed by the user to complete for example a running pattern, a sequence of actions, etc..

According to an optional aspect of the system, the system further comprises a system controller operationally coupled to the plurality of devices, the controller comprising:
- a directions retrieving unit, adapted to retrieve available directions of the plurality of devices;
- a direction selecting unit, adapted to select a direction from the available directions; and
- a direction informing unit, adapted to inform the direction selected.

A single controller hence may control plural devices. The system controller may select the direction randomly, or may be fed a sequence of stimuli, or may select the direction according to a predetermined sequence of stimuli. The predetermined sequence of stimuli may be manually implemented in the controller by for example a user of a device and/or a trainer of a user of a device, through for example a computer, or a tablet, or a smartphone, or a swatch, etc.. Alternatively, the predetermined sequence of stimuli may be retrieved from a storing unit of the system controller, wherein the storing unit is adapted to store a plurality of predetermined sequence of stimuli.

According to third aspect of the invention, there is provided a method for exercising reaction to stimuli as claimed in claim 17.

The method according to the present invention only generates a stimulus when presence is detected by a proximity sensor. A user of the method can therefore accurately train his reaction to a stimulus as the user himself triggers the generation of the stimulus when his presence is detected in the proximal sensing range of a proximity sensor. The user therefore does not have to identify which indicator generates a stimulus, thereby ensuring that the real reaction time of the user is measured. Additionally, each respective stimulus is indicative for a respective direction. Upon detection of presence, only one of the indicators generates a stimulus. In other words, each stimulus corresponds to the indication of one respective direction that a person detected in the proximal sensing range of the proximity sensor must follow. The method according to the present invention therefore allows a user to train a specific physical movement required to follow the respective direction associated with the stimulus. A direction is then a direction along which the user must move, for example by walking, or running, or crawling, etc., i.e. translation movements in a plane parallel to the ground. This is for example useful to footballers, basketballers, athletes in athletics, etc. who must train an unexpected change of direction when running to for example defend against an opponent, to get to the ball, or who must train their reaction to a starting pistol, etc.. Alternatively, a direction is then a direction along which the user must exercise a physical movement, for example by walking, or running, or jumping or bending, i.e. translation movements in a plane parallel to the vertical wall. This is for example useful to basketballers, athletes in athletics, swimmers, etc. who must train an unexpected change of direction when running to defend against an opponent, to get to the ball, or who must train their reaction to a starting pistol, etc.. The method according to the present invention hence generates a stimulus that must trigger a particular reaction from the user. The method hence enables to discriminate between correct and incorrect reaction to stimuli.

### Brief Description of the Drawings

Fig. 1 schematically illustrates an embodiment of a device according to the present invention.
Fig. 2 schematically illustrates an embodiment of a system according to the present invention, deployed on a football field.
Fig. 3 schematically illustrates an embodiment of a system according to the present invention, deployed on a basketball field.
Fig. 4 schematically illustrates an embodiment of a device according to the present invention used to train the reaction of a driver of a car.
Fig. 5 schematically illustrates an embodiment of a controller of a system according to the present invention.

### Detailed Description of Embodiment(s)

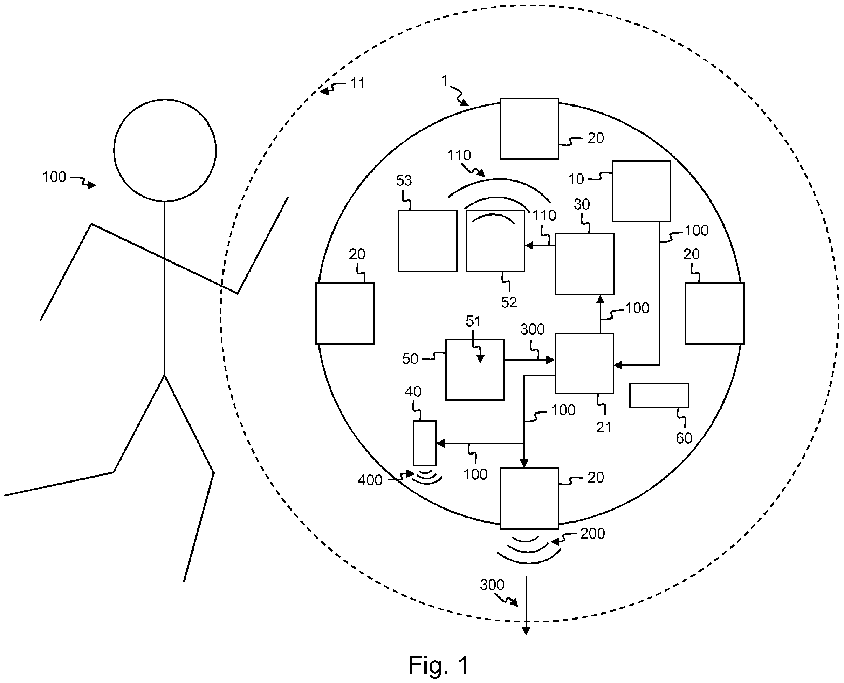

According to an embodiment shown in Fig. 1, a device 1 for exercising reaction to stimuli 200 comprises a proximity sensor 10, a controller 21, a reaction sensor 30, an auditory signal generator 40, a wireless receiver 50, a wireless transmitter 51, a wireless transceiver 53, a battery 60 and four indicators 20. The device 1 is round-shaped. According to an alternative embodiment, the device 1 is square-shaped, triangular-shaped, hexagonal-shaped, octagonal-shaped, etc.. The four indicators 20 are positioned at the edges of the device 1 and are equidistant. According to an alternative embodiment, the four indicators 20 are not equidistant. The proximity sensor 10 detects presence 100 in a proximal sensing range 11 of the proximity sensor 10. Presence 100 is human presence 100 detected by for example an infra-red proximity sensor 10. Fig. 1 is not at scale, the proximal sensing range 11 for example extending 10 meters, 2 meters, or 1 meter, or 50 centimetres, or 40 centimetres, or 30 centimetres from the proximity sensor 10. Each indicator 20 of the device 1 is indicative for a direction for presence 100, as depicted in Fig. 1, a right direction for presence 100, a left direction for presence 100, a straightforward direction for presence 100 and a backward direction for presence 100. Upon detection of presence 100 in the proximal sensing range 11 of the proximity sensor 10, the controller 21 instructs one of the four indicators 20 that presence 100 has been detected, thereby instructing the indicator 20 to generate a respective stimulus 200 indicative for a respective direction 300. The indicator 20 therefore generates a respective stimulus 200 indicative for a respective direction 300, i.e. a right direction for presence 100 as depicted in Fig. 1. The reaction sensor 30 detects a movement along the respective direction 300 and determines a reaction time 110 between generation of the respective stimulus 200 and detection of the movement along the respective direction 300. According to an alternative embodiment, the proximity sensor 10 and the reaction sensor 30 are integrated. The auditory signal generator 40 generates an auditory stimulus 400 indicative for the respective direction 300 upon detection of presence 100. The wireless receiver 50 receives signals 51 indicative for the respective direction 300 and feeds the respective direction 300 to the controller 21 which instructs the indicator 20 to generate the stimulus 200 indicative for the respective direction 300. The signals 51 are random. According to an alternative embodiment, the signals 51 follow a predetermined pattern of available directions. The reaction sensor 30 communicates the reaction time 110 to the wireless transmitter 52. The wireless transmitter 52 transmits the reaction time 110. The wireless transceiver 53 connects the device 1 with other similar devices and thereby determines a relative position of the device 1 versus other devices.

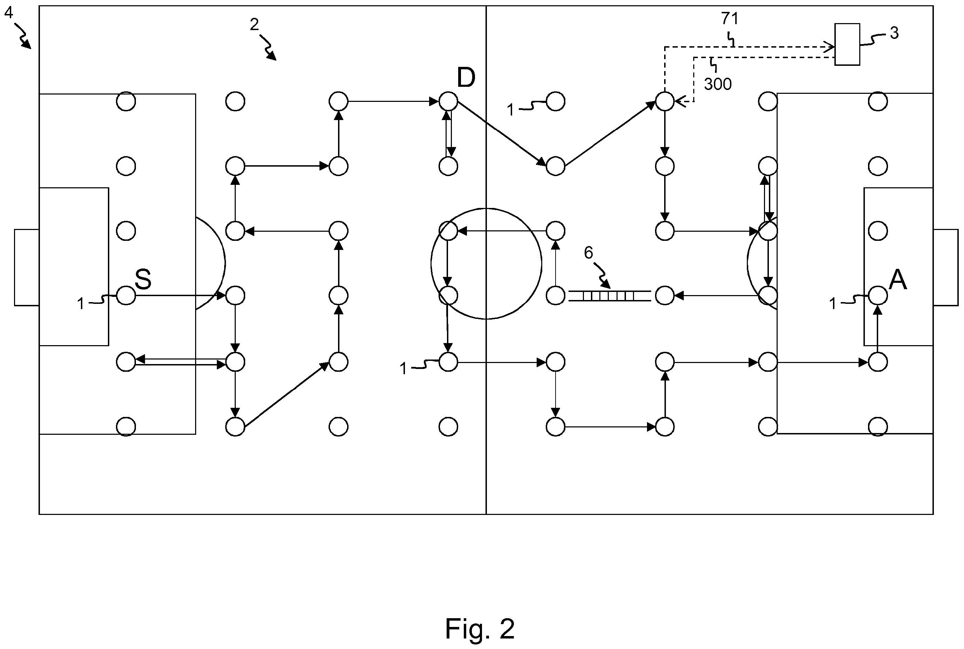

According to an embodiment shown in Fig. 2, a system 2 is deployed on a football field 4. The system 2 comprises forty-eight similar devices 1 deployed equidistantly on the football field 4, and a system controller 3. The devices 1 are aligned on the football field 4 in order to form a grid. According to an alternative embodiment, the devices 1 are arranged non-equidistantly. Each device 1 comprises a proximity sensor which detects presence in a proximal sensing range of the proximity sensor, and two or more indicators, each indicator being configured to generate a respective stimulus indicative for a respective direction. The controller 3 retrieves available directions 71 of the forty-eight similar devices 1. In order to keep Fig. 2 clear, the controller 3 retrieves available directions 71 for one device 1, but the controller 3 retrieves available directions 71 of the forty-eight similar devices 1. The controller 3 selects a direction from the available directions 71 and instructs one of the indicators of the devices 1 to generate the respective stimulus for the respective direction upon detection of presence. Not all the devices 1 of the system 2 are instructed to generate a stimulus indicative for a direction. According to an alternative embodiment, all the devices 1 of the system 2 are instructed to generate a stimulus. A user of the system 2 for example starts his training at the device 1 labelled S on Fig. 2. The user must then move along the respective directions indicated by one indicator of each device 1 of the system 2, thereby completing the path indicated on Fig. 2 by the arrows. The user thereby trains a specific movement as reaction to the stimuli generated by the system 2. Additionally, the training of Fig. 2 also comprises specific exercises, for example the ladder 6. The devices 1 of the system 2 generate a stimulus indicative for a left direction, a right direction, a straightforward direction, or a backward direction. The device 1 labelled D on Fig. 2 generates a stimulus indicative for a diagonal direction. The training of a user of the system 2 ends on device 1 labelled A which is not instructed to generate a stimulus and which therefore does not indicate a respective direction.

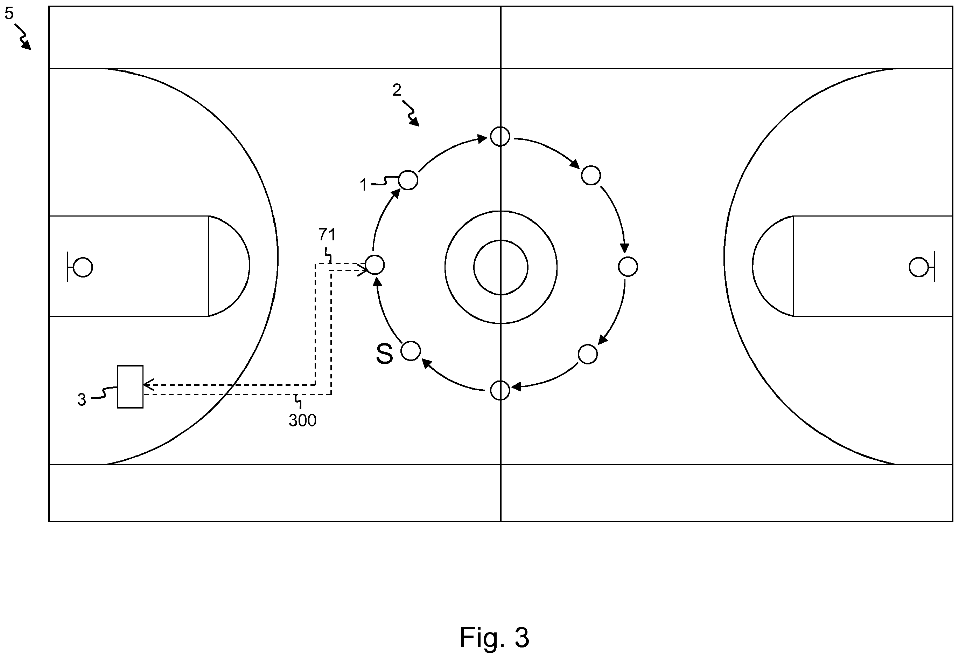

According to an embodiment shown in Fig. 3, a system 2 is deployed on a basketball field 5. The system 2 comprises eight similar devices 1 deployed equidistantly on the basketball field 5, and a system controller 3. The devices 1 are arranged on the basketball field 5 in the shape of a circle. According to an alternative embodiment, the devices 1 are arranged non-equidistantly. Each device 1 comprises a proximity sensor which detects presence in a proximal sensing range of the proximity sensor, and two or more indicators, each indicator being configured to generate a respective stimulus indicative for a respective direction. The controller 3 retrieves available directions 71 of the eight similar devices 1. In order to keep Fig. 3 clear, the controller 3 retrieves available directions 71 for one device 1, but the controller 3 retrieves available directions 71 of the eight similar devices 1. The controller 3 selects a direction from the available directions 71 and instructs one of the indicators of the devices 1 to generate the respective stimulus for the respective direction upon detection of presence. All the devices 1 of the system 2 are instructed to generate a stimulus indicative for a direction. According to an alternative embodiment, not all the devices 1 of the system 2 are instructed to generate a stimulus. A user of the system 2 for example starts his training at the device 1 labelled S on Fig. 3. The user must then move along the respective directions indicated by one indicator of each device 1 of the system 2, thereby completing the path indicated on Fig. 3 by the arrows. The user thereby trains a specific movement as reaction to the stimuli generated by the system 2. The devices 1 of the system 2 generate a stimulus indicative for a straightforward direction. According to an alternative embodiment, the devices 1 of the system 2 generate a stimulus indicative for a backward direction, or a left direction, or a right direction. The training of a user of the system 2 ends on device 1 labelled S which is not instructed to generate a consequent stimulus and which therefore does not indicate a respective direction.

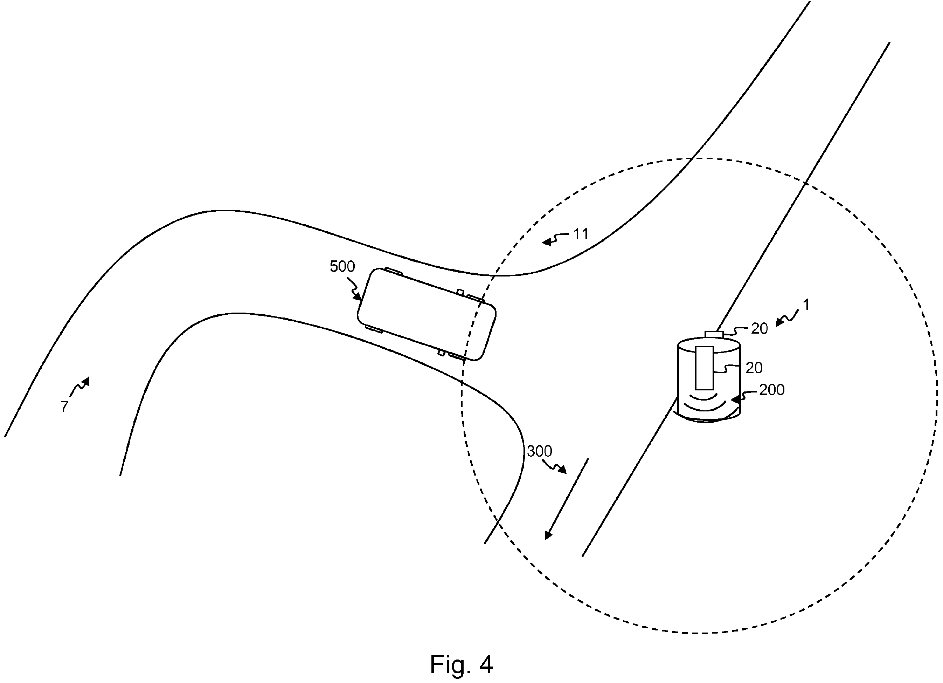

According to an embodiment shown in Fig. 4, a device 1 is positioned at a crossing of a road 7. The device 1 comprises a proximity sensor, which detects presence of a car 500, which is for example a rally car, in a proximal sensing range 11 of the proximity sensor. The device 1 comprises two indicators 20. Upon detection of presence of the car 500, one of the indicators 20 generates a stimulus 200 indicative for a respective direction 300 that the car 500 must follow. On Fig. 4, the car 500 is indicated that the respective direction 300 to follow at the crossing is to keep a right direction.

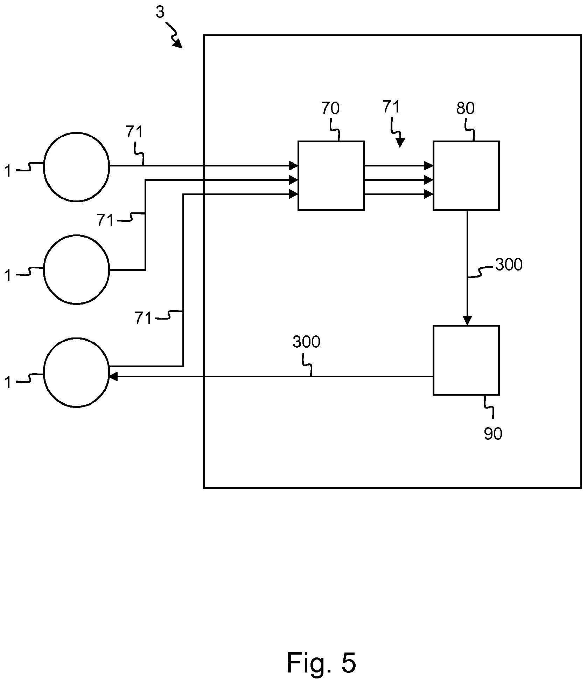

According to an embodiment shown in Fig. 5, a system controller 3 comprises a directions retrieving unit 70, a directions selecting unit 80 and a direction informing unit 90. The directions retrieving unit 70 retrieves available directions 71 of three similar devices 1. According to an alternative embodiment, the directions retrieving unit 70 retrieves available directions 71 from a plurality of devices 1. The directions selecting unit 80 selects a direction 300 from the available directions 71. The direction informing unit 90 informs one of the devices 1 on the direction 300 selected.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A device (1) for exercising reaction to stimuli (200), said device (1) comprising:
- a proximity sensor (10), adapted to detect presence (100) in a proximal sensing range (11) of said proximity sensor (10);
- two or more indicators (20), each indicator (20) being configured to generate a respective stimulus (200) indicative for a respective direction (300); wherein each respective stimulus (200) is generated to train a physical movement required to move along said respective direction (300) associated with said respective stimulus (200); and
- a controller (21) configured to instruct one of said two of more indicators (20) to generate said respective stimulus (200) for said respective direction (300) upon detection of said presence (100).

2. A device (1) according to claim 1, wherein said device (1) further comprises a reaction sensor (30) adapted to:
- detect movement along said respective direction (300);
- determine a reaction time (110) between generation of said respective stimulus (200) and detection of said movement along said respective direction (300).

3. A device (1) according to claim 2, wherein said proximity sensor (10) and said reaction sensor (30) are integrated.

4. A device (1) according to claims 1 to 3, wherein said device (1) comprises three indicators (20), respectively indicative for a left direction, a right direction and a straightforward direction.

5. A device (1) according to claims 1 to 3, wherein said device (1) comprises four indicators (20), respectively indicative for a left direction, a right direction, a straightforward direction and a backward direction.

6. A device (1) according to any of the preceding claims, wherein said indicators (20) are light-emitting diodes.

7. A device (1) according to claim 6, wherein to said respective direction (300) is associated one colour of said light-emitting diodes.

8. A device (1) according to any of the preceding claims, wherein said device (1) further comprises an auditory signal generator (40), being configured to generate an auditory stimulus (400) indicative for said respective direction (300) upon detection of said presence (100).

9. A device (1) according to any of the preceding claims, wherein said device (1) further comprises a wireless receiver (50) adapted to receive signals (51) indicative for said respective direction (300).

10. A device (1) according to claim 9, wherein said signals (51) are randomly alternating between available directions.

11. A device (1) according to claim 9, wherein said signals (51) follow a predetermined pattern of available directions.

12. A device (1) according to claim 9, wherein said device further comprises a wireless transmitter (52) further adapted to transmit said reaction time (110).

13. A device (1) according to any of the preceding claims, wherein said device further comprises a wireless transceiver (53) configured to connect said device (1) with other similar devices (1), and to determine a relative position of said device (1) versus said other devices (1).

14. A device (1) according to any of the preceding claims, wherein said device (1) further comprises a battery (60).

15. A system (2) for exercising reaction to stimuli (200), said system (2) comprising a plurality of devices (1) according to any of the preceding claims.

16. A system (2) according to claim 15, wherein said system (2) further comprises a system controller (3) operationally coupled to said plurality of devices (1), said controller (3) comprising:
- a directions retrieving unit (70), adapted to retrieve available directions (71) of said plurality of devices (1);
- a direction selecting unit (80), adapted to select a direction (300) from said available directions (71); and
- a direction informing unit (90), adapted to inform said device (1) on said direction (300) selected.

17. A method for exercising reaction to stimuli (200), said method comprising the steps of: - providing the device according to claim 1;
- detecting presence (100) in a proximal sensing range (11) by a proximity sensor (10) comprised in the device (1);
- generating in said device (1) upon detection of said presence a stimulus (200) indicative for a respective direction (300), said stimulus (100) being selected from a plurality of stimuli indicative for plural respective directions (300); and wherein each respective stimulus (200) is generated to train a physical movement required to move along said respective direction (300) associated with said respective stimulus (200).

## Patentansprüche

1. Vorrichtung (1) zum Ausüben von Reaktion auf Stimuli (200), wobei die Vorrichtung (1) Folgendes umfasst:
- einen Näherungssensor (10), der ausgelegt ist, um Vorhandensein (100) in einem proximalen Erfassungsbereich (11) des Näherungssensors (10) zu erfassen;
- zwei oder mehr Indikatoren (20), wobei jeder Indikator (20) konfiguriert ist, um einen jeweiligen Stimulus (200) zu erzeugen, der eine jeweilige Richtung (300) angibt; wobei jeder jeweilige Stimulus (200) erzeugt wird, um eine physische Bewegung zu trainieren, die erforderlich ist, um sich entlang der jeweiligen Richtung (300) zu bewegen, die mit dem jeweiligen Stimulus (200) verbunden ist; und
- eine Steuerung (21), die konfiguriert ist, um einen von den zwei oder mehr Indikatoren (20) anzuweisen, den jeweiligen Stimulus (200) für die jeweilige Richtung (300) bei Erfassung des Vorhandenseins (100) zu erzeugen.

2. Vorrichtung (1) nach Anspruch 1, wobei die Vorrichtung (1) ferner einen Reaktionssensor (30) umfasst, der für Folgendes ausgelegt ist:
- Erfassen von Bewegung entlang der jeweiligen Richtung (300);
- Bestimmen einer Reaktionszeit (110) zwischen der Erzeugung des jeweiligen Stimulus (200) und der Erfassung der Bewegung entlang der jeweiligen Richtung (300).

3. Vorrichtung (1) nach Anspruch 2, wobei der Näherungssensor (10) und der Reaktionssensor (30) integriert sind.

4. Vorrichtung (1) nach Anspruch 1 bis 3, wobei die Vorrichtung (1) drei Indikatoren (20) umfasst, die jeweils eine linke Richtung, eine rechte Richtung und eine Richtung geradeaus angeben.

5. Vorrichtung (1) nach Anspruch 1 bis 3, wobei die Vorrichtung (1) vier Indikatoren (20) umfasst, die jeweils eine linke Richtung, eine rechte Richtung, eine Richtung geradeaus und eine Rückwärtsrichtung angeben.

6. Vorrichtung (1) nach einem der vorhergehenden Ansprüche, wobei die Indikatoren (20) Leuchtdioden sind.

7. Vorrichtung (1) nach Anspruch 6, wobei mit der jeweiligen Richtung (300) eine Farbe der Leuchtdioden verbunden ist.

8. Vorrichtung (1) nach einem der vorhergehenden Ansprüche, wobei die Vorrichtung (1) ferner einen Akustiksignalgenerator (40) umfasst, der konfiguriert ist, um bei Erfassen des Vorhandenseins (100) einen akustischen Stimulus (400) zu erzeugen, der die jeweilige Richtung (300) angibt.

9. Vorrichtung (1) nach einem der vorhergehenden Ansprüche, wobei die Vorrichtung (1) ferner einen drahtlosen Empfänger (50) umfasst, der ausgelegt ist, um Signale (51) zu empfangen, die die jeweilige Richtung (300) angeben.

10. Vorrichtung (1) nach Anspruch 9, wobei die Signale (51) willkürlich zwischen verfügbaren Richtungen alternieren.

11. Vorrichtung (1) nach Anspruch 9, wobei die Signale (51) einem vorbestimmten Muster verfügbarer Richtungen folgen.

12. Vorrichtung (1) nach Anspruch 9, wobei die Vorrichtung ferner einen drahtlosen Sender (52) umfasst, der ferner ausgelegt ist, um die Reaktionszeit (110) zu übertragen.

13. Vorrichtung (1) nach einem der vorhergehenden Ansprüche, wobei die Vorrichtung ferner einen drahtlosen Sendeempfänger (53) umfasst, der konfiguriert ist, um die Vorrichtung (1) mit anderen ähnlichen Vorrichtungen (1) zu verbinden und um eine relative Position der Vorrichtung (1) gegenüber den anderen Vorrichtungen (1) zu bestimmen.

14. Vorrichtung (1) nach einem der vorhergehenden Ansprüche, wobei die Vorrichtung (1) ferner eine Batterie (60) umfasst.

15. System (2) zum Ausüben von Reaktion auf Stimuli (200), wobei das System (2) eine Vielzahl von Vorrichtungen (1) nach einem der vorhergehenden Ansprüche umfasst.

16. System (2) nach Anspruch 15, wobei das System (2) ferner eine Systemsteuerung (3) umfasst, die an die Vielzahl von Vorrichtungen (1) wirkgekoppelt ist, wobei die Steuerung (3) Folgendes umfasst:
- eine Richtungsabrufeinheit (70), die ausgelegt ist, um verfügbare Richtungen (71) der Vielzahl von Vorrichtungen (1) abzurufen;
- eine Richtungsauswahleinheit (80), die ausgelegt ist, um eine Richtung (300) aus den verfügbaren Richtungen (71) auszuwählen; und
- eine Richtungsinformiereinheit (90), die ausgelegt ist, um die Vorrichtung (1) über die ausgewählte Richtung (300) zu informieren.

17. Verfahren zum Ausüben von Reaktion auf Stimuli (200), wobei das Verfahren die folgenden Schritte umfasst:
- Bereitstellen der Vorrichtung nach Anspruch 1;
- Erfassen von Vorhandensein (100) in einem proximalen Erfassungsbereich (11) durch einen Näherungssensor (10), der in der Vorrichtung (1) enthalten ist;
- Erzeugen eines Stimulus (200) in der Vorrichtung (1) bei Erfassen des Vorhandenseins, der eine jeweilige Richtung (300) angibt, wobei der Stimulus (100) aus einer Vielzahl von Stimuli ausgewählt ist, die mehrere jeweilige Richtungen (300) angeben; und wobei jeder jeweilige Stimulus (200) erzeugt wird, um eine physische Bewegung zu trainieren, die erforderlich ist, um sich entlang der jeweiligen Richtung (300) zu bewegen, die mit dem jeweiligen Stimulus (200) verbunden ist.

## Revendications

1. Dispositif (1) destiné à exercer une réaction à des stimuli (200), ledit dispositif (1) comprenant :
- un capteur de proximité (10), adapté pour détecter une présence (100) dans une plage de détection proximale (11) dudit capteur de proximité (10) ;
- deux indicateurs (20), ou plus, chaque indicateur (20) étant configuré pour générer un stimulus respectif (200) indiquant une direction respective (300) ; chaque stimulus respectif (200) étant généré pour entraîner un mouvement physique nécessaire pour se déplacer dans ladite direction respective (300) associée audit stimulus respectif (200) ; et
- un dispositif de commande (21) conçu pour commander un indicateur desdits deux indicateurs parmi plusieurs indicateurs (20) de générer ledit stimulus respectif (200) pour ladite direction respective (300) lors de la détection de ladite présence (100).

2. Dispositif (1) selon la revendication 1, ledit dispositif (1) comprenant en outre un capteur de réaction (30) adapté pour :
- détecter un mouvement le long ladite direction respective (300) ;
- déterminer un temps de réaction (110) entre la génération dudit stimulus respectif (200) et la détection dudit mouvement le long de ladite direction respective (300).

3. Dispositif (1) selon la revendication 2, ledit capteur de proximité (10) et ledit capteur de réaction (30) étant intégrés.

4. Dispositif (1) selon les revendications 1 à 3, ledit dispositif (1) comprenant trois indicateurs (20), indiquant respectivement une direction gauche, une direction droite et une direction avant.

5. Dispositif (1) selon les revendications 1 à 3, ledit dispositif (1) comprenant quatre indicateurs (20), indiquant respectivement une direction gauche, une direction droite, une direction avant et une direction arrière.

6. Dispositif (1) selon l'une quelconque des revendications précédentes, lesdits indicateurs (20) étant des diodes électroluminescentes.

7. Dispositif (1) selon la revendication 6, une couleur desdites diodes électroluminescentes étant associée à ladite direction respective (300).

8. Dispositif (1) selon l'une quelconque des revendications précédentes, ledit dispositif (1) comprenant en outre un générateur de signal auditif (40) configuré pour générer un stimulus auditif (400) indiquant ladite direction respective (300) lors de la détection de ladite présence (100).

9. Dispositif (1) selon l'une quelconque des revendications précédentes, ledit dispositif (1) comprenant en outre un récepteur sans fil (50) adapté pour recevoir des signaux (51) indiquant ladite direction respective (300).

10. Dispositif (1) selon la revendication 9, lesdits signaux (51) étant alternés aléatoirement entre des directions disponibles.

11. Dispositif (1) selon la revendication 9, lesdits signaux (51) suivant un motif préétabli de directions disponibles.

12. Dispositif (1) selon la revendication 9, ledit dispositif comprenant en outre un transmetteur sans fil (52) adapté en outre pour transmettre ledit temps de réaction (110).

13. Dispositif (1) selon l'une quelconque des revendications précédentes, ledit dispositif comprenant en outre un émetteur-récepteur sans fil (53) conçu pour connecter ledit dispositif (1) à d'autres dispositifs similaires (1) et pour déterminer une position relative dudit dispositif (1) par rapport aux autres dispositifs (1).

14. Dispositif (1) selon l'une quelconque des revendications précédentes, ledit dispositif (1) comprenant en outre une batterie (60).

15. Système (2) destiné à exercer une réaction à des stimuli (200), ledit système (2) comprenant une pluralité de dispositifs (1) selon l'une quelconque des revendications précédentes.

16. Système (2) selon la revendication 15, ledit système (2) comprenant en outre un dispositif de commande (3) de système couplé de manière fonctionnelle à ladite pluralité de dispositifs (1), ledit dispositif de commande (3) comprenant :
- une unité de récupération de directions (70), adaptée pour récupérer des directions disponibles (71) de ladite pluralité de dispositifs (1) ;
- une unité de sélection de direction (80), adaptée pour sélectionner une direction (300) parmi lesdites directions disponibles (71) ; et
- une unité d'information de direction (90), adaptée pour informer ledit dispositif (1) quant à ladite direction (300) sélectionnée.

17. Procédé permettant d'exercer une réaction à des stimuli (200), ledit procédé comprenant les étapes de :
- obtention du dispositif selon la revendication 1 ;
- détection d'une présence (100) dans une plage de détection proximale (11) par un capteur de proximité (10) compris dans le dispositif (1) ;
- génération dans ledit dispositif (1), lors de la détection de ladite présence d'un stimulus (200) indiquant une direction respective (300), ledit stimulus (100) étant sélectionné parmi une pluralité de stimuli indiquant plusieurs directions respectives (300) ; et chaque stimulus respectif (200) étant généré pour entraîner un mouvement physique nécessaire pour se déplacer dans ladite direction respective (300) associée audit stimulus respectif (200).
